# FASCICULE DE BREVET EUROPEEN

(11) **EP 2 440 510 B1**
(45) Date de publication et mention de la délivrance du brevet: **27.04.2016**
(21) Numéro de dépôt: 10727461.5
(22) Date de dépôt: 06.05.2010
(51) Int. Cl.: C07C 17/354, C07C 19/08

(54) **PROCEDE FABRICATION DU PENTAFLUOROPROPANE**
VERFAHREN ZUR HERSTELLUNG VON PENTAFLUORPROPAN
METHOD FOR PRODUCING PENTAFLUOROPROPANE

(30) Priorité: 12.06.2009 FR 0953937
(43) Date de publication de la demande: 18.04.2012
(73) Titulaire: ARKEMA FRANCE, 92700 Colombes (FR)
(72) Inventeur: DEVIC, Michel, 69110 Sainte Foy Les Lyon (FR); DOUCET, Nicolas, 69007 Lyon (FR); WENDLINGER, Laurent, 69150 Soucieu En Jarrest (FR); CAVALLINI, Géraldine, 69360 Saint-Symphorien D'ozon (FR)
(74) Mandataire: Dang, Doris
(86) Numéro de dépôt international: PCT/FR2010/050866
(87) Numéro de publication internationale: WO 2010/142878

(56) Documents cités:
- EP-A1- 0 644 173
- EP-A1- 0 726 243
- EP-A1- 1 916 232
- WO-A2-2008/030440
- DATABASE WPI Week 199401 Thomson Scientific, London, GB; AN 1994-007408 XP002564216 & WO 93/25510 A1 (DAIKIN IND LTD) 23 décembre 1993 (1993-12-23)
- DATABASE CA [Online] CHEMICAL ABSTRACTS SERVICE, COLUMBUS, OHIO, US; 1966, SIANESI, DARIO ET AL: "Fluoro olefins. I. cis- and trans-1,2,3,3,3-Pentafluoropropylene" XP002564152 extrait de STN Database accession no. 1966:3555 & ANN. CHIM (ROME) , 55(8-9), 850-61, 1965 ,
- DATABASE CA [Online] CHEMICAL ABSTRACTS SERVICE, COLUMBUS, OHIO, US; 1961, KNUNYANTS, I. L. ET AL: "Reactions of fluoro olefins. XIII. Catalytic hydrogenation of perfluoro olefins" XP002564154 extrait de STN Database accession no. 1961:2127 cité dans la demande & IZVESTIYA AKADEMII NAUK SSSR, SERIYA KHIMICHESKAYA 1412-18 CODEN: IASKA6; ISSN: 0002-3353, 1960,

## Description

La présente invention concerne un procédé de fabrication du 1,1,1,2,3-pentafluoropropane par hydrogénation du 1,2,3,3,3-pentafluoropropène.

Le 2,3,3,3-tetrafluoropropène est connu pour ses propriétés de réfrigérants et fluides caloporteurs. Le procédé de fabrication du 2,3,3,3-tetrafluoropropène à partir du 1,2,3,3,3-pentafluoropropène comprend une étape d'hydrogénation du 1,2,3,3,3-pentafluoropropène.

Le document de Knunyants et al., Journal of the USSR Academy of Sciences, Chemistry Department, «reactions of fluoro-olefins», report 13, «catalytic hydrogénation of perfluoro-olefins», 1960, décrit l'hydrogénation du 1,2,3,3,3-pentafluoropropène (HFO-1225ye) à température ambiante sur un catalyseur palladium supporté sur alumine pour donner un mélange de 1,1,1,2,3-pentafluoropropane (HFC-245eb) et de 1,1,1,2-tetrafluoropropane (HFC-254eb). Le 1,1,1,2-tetrafluoropropane est produit en quantité significative (c-à-d environ 50% par rapport au 1,1,1,2,3-pentafluoropropane).

Le document WO 2008/030440 décrit une méthode de préparation du 2,3,3,3-tetrafluoropropène comprenant au moins une étape d'hydrogénation au cours de laquelle du 1,2,3,3,3-pentafluoropropène est mis en contact avec de l'hydrogène en présence d'un catalyseur. D'après ce document, le catalyseur d'hydrogénation pouvant convenir contient un métal du groupe VIII ou le rhénium et le métal peut être supporté.

L'exemple 1 du document WO 2008/030440 décrit la réaction d'hydrogénation du 1,2,3,3,3-pentatluoropropène à 85°C en présence d'un catalyseur contenant 0,5% en poids de palladium supporté sur du charbon pour donner un flux contenant 92% du HFC-245eb et 8% de HFC-254eb.

Les essais de l'art antérieur précité ont été effectués à l'échelle laboratoire et les documents sont totalement muets sur la durée de vie de ces catalyseurs. Les réactions d'hydrogénation telles que décrites précédemment sont des réactions fortement exothermiques et posent des problèmes à l'échelle industrielle. En outre, il se forme une quantité non négligeable de sous produit (HFC-254eb) due probablement à la réaction consécutive d'hydrogénolyse du HFC-245eb (c-à-d la substitution d'un atome de fluor du produit recherché par un atome d'hydrogène avec formation d'acide fluorhydrique).
La présence d'un composé, autre que les réactifs, dans le flux réactionnel peut également être à l'origine d'une désactivation rapide du catalyseur.

EP 726243 décrit un procédé de fabrication du 1,2,3,3,3-pentafluoropropène en faisant réagir du 1,1,1,2,3- hexafluoropropane avec de l'HF en phase gazeuse en présence d'un oxyde de chrome trivalent ou d'un oxyde de chrome trivalent partiellement fluoré.

EP 644173 décrit un procédé de fabrication du 1,2,3,3,3-pentafluoropropène en faisant réagir du 1,1,1,2,3- hexafluoropropane avec de l'HF en présence d'un catalyseur d'hydrogénation comprenant du palladium et des dopants métalliques ou du rhodium.

Par ailleurs, le document EP 1916232 propose une réaction d'hydrogénation multiétagée d'un composé oléfinique pour obtenir une conversion et une sélectivité élevée. L'exemple 2 décrit l'hydrogénation étagée du 1,2,3,3,3-pentafluoropropène en présence d'un catalyseur de palladium supporté sur du charbon dans quatre réacteurs avec une température de sortie du premier réacteur de 99°C, une température de sortie du deuxième réacteur de 95°C pour une conversion de 54%, une température de 173°C à la sortie du troisième réacteur et une température de 104°C à la sortie du quatrième réacteur. Il est prévu des étapes de refroidissement entre les réacteurs avec une température du premier bain de 59°C et une température du deuxième bain de 116°C.

Le procédé tel que décrit dans le document EP 1916232 est coûteux et en outre, sa mise en oeuvre n'est pas aisée.

La présente demande fournit un procédé de fabrication continu ou semi-continu du 1,1,1,2,3-pentafluoropropane à partir du 1,2,3,3,3-pentafluoropropène qui permet de résoudre en partie ou en totalité les inconvénients précités.

Le procédé, selon la présente invention, permet plus particulièrement de maîtriser l'exothermicité de la réaction d'hydrogénation et/ou de limiter la réaction d'hydrogénolyse du HFC-245eb et/ou de réduire la désactivation du catalyseur.

Le procédé selon la présente invention est caractérisé en ce que (i) l'on fait réagir en phase gazeuse du 1,2,3,3,3-pentafluoropropène avec de l'hydrogène en quantité surstoechiométrique, à une température comprise entre 80 et 250 °C, de préférence comprise entre 110 et 160°C, en présence d'un catalyseur d'hydrogénation dans un réacteur ; (ii) l'on recycle une partie de l'effluent gazeux issu du réacteur comprenant du 1,1,1,2,3-pentafluoropropane, de l'hydrogène non réagi , éventuellement du 1,2,3,3,3-pentafluoropropène non réagi, du 1,1,1,2-tetrafluoropropane et de l'acide fluorhydrique et (iii) l'on récupère le 1,2,3,3,3-pentafluoropropane de l'autre partie de l'effluent issu du réacteur, éventuellement après une étape de purification.

De préférence, la température à l'entrée du lit catalytique est comprise entre 50 et 200°C, avantageusement comprise entre 80 et 140°C.

Le flux recyclé au réacteur ainsi que les réactifs peuvent être préchauffés avant l'introduction dans le réacteur.

Le procédé selon la présente invention est de préférence mis en oeuvre avec un rapport molaire hydrogène / HFO-1225ye compris entre 1,2 et 40, avantageusement compris entre 3 et 10. Ce rapport est en général obtenu par ajout du 1,2,3,3,3-pentafluoropropène et d'hydrogène au flux de recyclage.

Le temps de contact, défini comme le rapport du volume du lit catalytique sur le débit volumique du flux total dans les conditions normales de température et de pression, est de préférence comprise entre 0,1s et 20s et avantageusement comprise entre 0,5 et 5s.

La réaction d'hydrogénation, selon la présente invention, est de préférence mise en oeuvre à une pression comprise entre 0,5 et 20 bar absolu et avantageusement comprise entre 1 et 5 bar absolu.

L'effluent gazeux à la sortie du réacteur comprend de préférence de 5 à 96 % en volume du 1,1,1,2,3-pentafluoropropane, 2 à 90% en volume d'hydrogène, de 1 à 20% en 1,1,1,2-tetrafluoropropane, et de 0 à 10% du 1,1,1,2,3-pentafluoropropène.

Avantageusement, l'effluent gazeux à la sortie du réacteur comprend de 5 à 91 % en volume du 1,1,1,2,3-pentafluoropropane, de 8 à 50% en volume d'hydrogène, de 1 à 5 % en volume de 1,1,1,2-tetrafluoropropane et de 0 à 0,1 % en volume du 1,1,1,2,3-pentafluoropropène.

Selon le procédé de la présente invention, on utilise de préférence un réacteur adiabatique.

La partie de l'effluent gazeux qui est recyclée au réacteur représente, de préférence au moins 80% en volume de la totalité de l'effluent à la sortie du réacteur, avantageusement au moins 90% en volume. De façon particulièrement préférée, la partie de l'effluent recyclée au réacteur représente entre 93 et 98% en volume de l'effluent total à la sortie du réacteur.

Comme catalyseur, on peut notamment citer ceux à base d'un métal du groupe VIII ou rhénium. Le catalyseur peut être supporté, par exemple sur carbone, alumine, fluorure d'aluminium, etc. ou peut ne pas être supporté, comme le nickel de Raney. Comme métal, on peut utiliser du platine ou du palladium, en particulier du palladium, avantageusement supporté sur carbone ou alumine. On peut aussi associer ce métal avec un autre métal comme l'argent, le cuivre, l'or, le tellure, le zinc, le chrome, le molybdène et le thallium.

Le catalyseur préféré comprend du palladium éventuellement supporté. Le catalyseur tout particulièrement préféré selon la présente invention est un catalyseur contenant du palladium sur un support à base d'alumine. La quantité de palladium dans le catalyseur est de préférence comprise entre 0,05 et 10% en poids et avantageusement entre 0,1 et 5%.

La surface spécifique du catalyseur est de préférence supérieure à 4 m²/g. L'alumine utilisée en tant que support catalytique se présente avantageusement sous la forme polymorphique α.

La demanderesse a remarqué de façon surprenante que la quantité du sous produit HFC-254eb reste faible malgré le recyclage d'une partie de l'effluent gazeux à la sortie du réacteur. Cette quantité est même inférieure par rapport à l'art antérieur en l'absence de recyclage.
Le procédé selon la présente invention permet d'obtenir une conversion du HFO-1225ye élevée et une sélectivité en HFC-245eb élevée. Ces performances sont en outre stables dans le temps. Ceci permet de limiter la présence de l'acide fluorhydrique (produit très corrosif) dans la boucle de recyclage.

### PARTIE EXPERIMENTALE

Les essais suivants ont été effectués avec un dispositif permettant de recycler une partie de l'effluent au réacteur.

La conversion est définie comme étant le pourcentage de HFO-1225ye qui est transformé.
La sélectivité en produit X est définie comme étant le pourcentage du nombre de moles de produit X formé sur le nombre de moles de HFO-1225ye transformé.

### Exemple 1 :

On utilise un réacteur tubulaire en inox de diamètre interne 2,1 cm et de longueur 120 cm contenant 469 g soit 320 cm3 de catalyseur sous forme d'un lit fixe. Le catalyseur contient 0,2 % en poids de palladium supporté sur alumine α.
Pendant la durée de la réaction, on injecte en continu 1,41 mole/h d'hydrogène et 0,7 mole/h de 1,1,1,2,3-pentafluoropropène et le débit de la boucle de recyclage est de 0,490 Nm3, soit 93,7% en volume de l'effluent gazeux à la sortie du réacteur. Le rapport molaire de l'hydrogène/ HFO-1225ye à l'entrée du lit catalytique est de 16. La pression est de 1 bar absolu. La température à l'entrée du réacteur est de 60°C et la température de réacteur maximale atteinte en cours de réaction est de 124°C. Le temps de contact est de 2,3 secondes On obtient une conversion de 100% en HFO-1225ye, une sélectivité de 95,7% en HFC-245eb et une sélectivité de 4,1% en HFC-254eb.

Aucune désactivation n'a été observée pendant 80h de fonctionnement.

### Exemple 2 :

On utilise le même appareil que précédemment avec le même catalyseur. Pendant la durée de la réaction, on injecte en continu 0,84 mole/h d'hydrogène et 0,7 mole/h de 1,1,1,2,3-pentafluoropropène et le débit au sein de la boucle de recyclage est de 0,970 Nm3/h, soit un pourcentage volumique de recyclage de 98%. Le rapport molaire de l'hydrogène/ HFO-1225ye à l'entrée du réacteur est de 1,18. La pression est de 2 bar absolue. La température à l'entrée du lit catalytique est de 63°C et la température de réacteur maximale atteinte en cours de réaction est de 90°C. Le temps de contact est de 1,2 s.

On obtient une conversion de 100% en HFO-1225ye, une sélectivité de 79% en HFC-245eb et une sélectivité de 20,0% en HFC-254eb.

### Exemple 3

On opère dans les mêmes conditions que l'exemple 2 à l'exception que le rapport molaire de l'hydrogène/ HFO-1225ye à l'entrée du réacteur est de 5,2 et que la température à l'entrée du lit catalytique est de 100°C. La température maximale atteinte en cours de réaction est de 123°C.

On obtient une conversion de 100% en HFO-1225ye, une sélectivité de 89,6% en HFC-245eb et une sélectivité de 10,2% en HFC-254eb.

## Revendications

1. Procédé de fabrication du 1,2,3,3,3-pentafluoropropane **caractérisé en ce que** (i) l'on fait réagir en phase gazeuse du 1,2,3,3,3-pentafluoropropène avec de l'hydrogène en quantité surstoechiométrique, à une température comprise entre 80 et 250°C, de préférence comprise entre 110 et 160°C, en présence d'un catalyseur d'hydrogénation dans un réacteur; (ii) l'on recycle une partie de l'effluent gazeux issu du réacteur comprenant du 1,2,3,3,3-pentafluoropropane, de l'hydrogène non réagi éventuellement du 1,2,3,3,3-pentafluoropropène non réagi et du 1,1,1,2-tetrafluoropropane et de l'acide fluorhydrique et (iii) l'on récupère le 1,2,3,3,3-pentafluoropropane de l'autre partie de l'effluent issu du réacteur, éventuellement après une étape de purification.

2. Procédé selon la revendication 1 **caractérisé en ce que** la partie de l'effluent gazeux recyclé représente au moins 80%, de préférence entre 93 et 98% en volume de la totalité de l'effluent à la sortie du réacteur.

3. Procédé selon la revendication 1 ou 2 **caractérisé en ce que** l'effluent gazeux à la sortie du réacteur comprend de 5 à 96 % en volume du 1,1,1,2,3-pentafluoropropane, 2 à 90% en volume d'hydrogène, 1 à 20% en 1,1,1,2-tetrafluoropropane et 0 à 10% du 1,1,1,2,3-pentafluoropropène.

4. Procédé selon l'une quelconque des revendications précédentes **caractérisé en ce que** le catalyseur comprend du palladium, éventuellement supporté.

5. Procédé selon la revendication 4 **caractérisé en ce que** le support est à base d'alumine.

6. Procédé selon l'une quelconque des revendications précédentes **caractérisé en ce que** le rapport molaire H₂/1,2,3,3,3- pentafluoropropène est compris entre 1,5 et 40, de préférence compris entre 3 et 10.

7. Procédé selon l'une quelconque des revendications précédentes **caractérisé en ce que** le temps de contact est compris entre 0,2 s et 20 s et de préférence compris entre 1 et 5 s.

8. Procédé selon l'une quelconque des revendications précédentes **caractérisé en ce que** la réaction d'hydrogénation est mise en oeuvre à une pression comprise entre 0,5 et 20 bar absolu et de préférence comprise entre 1 et 5 bar absolu.

9. Procédé selon l'une quelconque des revendications précédentes **caractérisé en ce qu'**il est mis en oeuvre en continu.

## Patentansprüche

1. Verfahren zur Herstellung von 1,2,3,3,3-Pentafluorpropan, **dadurch gekennzeichnet, dass** man (i) 1,2,3,3,3-Pentafluorpropen in der Gasphase bei einer Temperatur zwischen 80 und 250°C und vorzugsweise zwischen 110 und 160°C in Gegenwart eines Hydrierkatalysators in einem Reaktor mit Wasserstoff in überstöchiometrischer Menge umsetzt; (ii) einen Teil des gasförmigen Austragsstroms aus dem Reaktor, der 1,2,3,3,3-Pentafluorpropan, nicht umgesetzten Wasserstoff, gegebenenfalls nicht umgesetztes 1,2,3,3,3-Pentafluorpropen und 1,1,1,2-Tetrafluorpropan und Fluorwasserstoffsäure umfasst, rezykliert und (iii) aus dem anderen Teil des Austragsstroms aus dem Reaktor 1,2,3,3,3-Pentafluorpropan gewinnt, gegebenenfalls nach einem Reinigungsschritt.

2. Verfahren nach Anspruch 1, **dadurch gekennzeichnet, dass** der rezyklierte Teil des gasförmigen Austragsstroms mindestens 80 Vol.-% und vorzugsweise zwischen 93 und 98 Vol.-% des gesamten Austragsstroms am Ausgang des Reaktors beträgt.

3. Verfahren nach Anspruch 1 oder 2, **dadurch gekennzeichnet, dass** der gasförmige Austragsstrom am Ausgang des Reaktors 5 bis 96 Vol.-% 1,1,1,2,3-Pentafluorpropan, 2 bis 90 Vol.-% Wasserstoff, 1 bis 20% 1,1,1,2-Tetrafluorpropan und 0 bis 10% 1,1,1,2,3-Pentafluorpropen umfasst.

4. Verfahren nach einem der vorhergehenden Ansprüche, **dadurch gekennzeichnet, dass** der Katalysator gegebenenfalls geträgertes Palladium umfasst.

5. Verfahren nach Anspruch 4, **dadurch gekennzeichnet, dass** der Träger auf Aluminiumoxid basiert.

6. Verfahren nach einem der vorhergehenden Ansprüche, **dadurch gekennzeichnet, dass** das Molverhältnis von H₂ zu 1,2,3,3,3-Pentafluorpropen zwischen 1,5 und 40 und vorzugsweise zwischen 3 und 10 liegt.

7. Verfahren nach einem der vorhergehenden Ansprüche, **dadurch gekennzeichnet, dass** die Kontaktzeit zwischen 0,2 s und 20 s und vorzugsweise zwischen 1 und 5 s liegt.

8. Verfahren nach einem der vorhergehenden Ansprüche, **dadurch gekennzeichnet, dass** die Hydrierungsreaktion bei einem Druck zwischen 0,5 und 20 bar absolut und vorzugsweise zwischen 1 und 5 bar absolut durchgeführt wird.

9. Verfahren nach einem der vorhergehenden Ansprüche, **dadurch gekennzeichnet, dass** es kontinuierlich durchgeführt wird.

## Claims

1. Process for the manufacture of 1,1,1,2,3-pentafluoropropane, **characterized in that** (i) 1,2,3,3,3-pentafluoropropene is reacted in the gas phase with hydrogen in a superstoichiometric amount, at a temperature of between 80 and 250°C, preferably of between 110 and 160°C, in the presence of a hydrogenation catalyst in a reactor; (ii) a portion of the gaseous output stream resulting from the reactor, comprising 1,1,1,2,3-pentafluoropropane, unreacted hydrogen, optionally unreacted 1,2,3,3,3-pentafluoropropene, 1,1,1,2-tetrafluoropropane and hydrofluoric acid, is recycled and (iii) the 1,1,1,2,3-pentafluoropropane is recovered from the other portion of the output stream resulting from the reactor, optionally after a purification stage.

2. Process according to Claim 1, **characterized in that** the portion of the gaseous output stream recycled represents at least 80%, preferably between 93 and 98%, by volume of the whole of the output stream at the outlet of the reactor.

3. Process according to Claim 1 or 2, **characterized in that** the gaseous output stream at the outlet of the reactor comprises from 5 to 96% by volume of the 1,1,1,2,3-pentafluoropropane, from 2 to 90% by volume of hydrogen, from 1 to 20% of 1,1,1,2-tetrafluoropropane and from 0 to 10% of the 1,2,3,3,3-pentafluoropropene.

4. Process according to any one of the preceding claims, **characterized in that** the catalyst comprises optionally supported palladium.

5. Process according to Claim 4, **characterized in that** the support is based on alumina.

6. Process according to any one of the preceding claims, **characterized in that** the H₂/1,2,3,3,3-pentafluoropropene molar ratio is between 1.5 and 40, preferably between 3 and 10.

7. Process according to any one of the preceding claims, **characterized in that** the contact time is between 0.2 and 20 s and preferably between 1 and 5 s.

8. Process according to any one of the preceding claims, **characterized in that** the hydrogenation reaction is carried out at a pressure of between 0.5 and 20 bar absolute and preferably of between 1 and 5 bar absolute.

9. Process according to any one of the preceding claims, **characterized in that** it is carried out continuously.
